# FASCICULE DE BREVET EUROPEEN

(11) **EP 0 518 756 B1**
(45) Date de publication et mention de la délivrance du brevet: **22.03.2000**
(21) Numéro de dépôt: 92401600.9
(22) Date de dépôt: 10.06.1992
(51) Int. Cl.: G01N 33/569, C07K 14/18, C12N 15/40, C12P 21/08, G01N 33/577

(54) **Procédé de détection de l'infection par le virus de la diarrhée bovine, séquence nucléotidique codant pour une protéine induite par l'infection par ce virus et protéines et antigènes recombinants s'y rapportant**
Verfahren zum Nachweis einer Rinderdiarrhoe- virusinfektion, für ein durch eine Infektion mit diesem Virus induziertes Eiweiss kodierende Nukleotidsequenz, sowie daraus abgeleitete rekombinante Eiweisse und Antigene
Procedure for the detection of a bovine viral diarrhoea virus infection, nucleotide sequence coding for a protein induced by infection with this virus, as well as recombinant proteins and antigens derived therefrom same

(30) Priorité: 11.06.1991 FR 9107076
(43) Date de publication de la demande: 16.12.1992
(73) Titulaire: MERIAL, 69002 Lyon (FR)
(72) Inventeur: Vandenbergh, Danielle, Hélène, Jeanne, B-4602 Cheratte (BE); Lecomte, Corine, Martine, Thérèse, Ghislaine, B-4560 Ocquier (BE); Chappuis, Gilles-Emile, F-69006 Lyon (FR); Pin, Jean-Jacques, F-69720 Saint Bonnet de Mure (FR)
(74) Mandataire: Bernasconi, Jean

(56) Documents cités:
- EP-A- 0 208 672
- VETERINARY MICROBIOLOGY vol. 23, 1990, AMSTERDAM NL pages 193 - 201; C. LECOMPTE ET AL: 'ELISA detection of bovine viral diarrhoea virus specific antibodies using recombinant antigen and monoclonal antibodies.'
- VIROLOGY vol. 184, no. 1, 1988, WASHINGTON DC USA pages 191 - 199; M.S. COLLET ET AL.: 'Molecular cloning and nucleotide sequence of the pestivirus bovine viral diarrhea virus.'
- BIOCHEMICAL AND BIOPHYSICAL RESEARCH COMMUNICATIONS vol. 178, no. 3, 15 Août 1991, NEW YORK NY USA pages 1326 - 1334; J. KWANG ET AL.: 'Expression of the p80 region of bovine viral diarrhea virus and identification of specific antibodies to this recombinant protein in bovine sera.'
- Résumé distribué au VIIIème Congrès international de virologie, Berlin, 26.-31.8.1990: C. Lecomte et al., "ELISA detection of pestivirus infection using recombinant antigen and monoclonal antibodies"

## Description

La présente invention concerne un procédé de détection de l'infection par le virus de la diarrhée bovine, une séquence nucléotidique codant pour une protéine associée à l'infection par ce virus et des protéines et antigènes recombinants s'y rapportant.

Le virus de la diarrhée bovine (BVD) est un virus enveloppé à ARN monocaténaire infectieux, qui est apparenté au virus de la peste porcine classique et à celui de la maladie de Border, les trois virus formant le genre Pestivirus qui appartient à la famille des Togaviridae. Le virus BVD est universellement répandu dans les populations bovines et se manifeste par un très large éventail de symptômes cliniques associés à des maladies congénitales, respiratoires ou entérigues (diarrhée virale bovine, maladie des muqueuses).

Les isolats des virus BVD peuvent être classés en deux catégories ou biotypes distincts d'après leurs effets en culture de cellules : cytopathogène et non cytopathogène.

L'infection aiguë d'animaux séronégatifs est ordinairement bénigne ou subclinique. Par contre, une infection intra-utérine du foetus, dans les quatre premiers mois environ après le début de la gestation, par une souche non cytopathogène, peut non seulement produire des avortements, des mortinatalités ou la naissance de veaux faibles, mais aussi la naissance de veaux présentant une virémie persistante, c'est-à-dire excrétant le virus de manière permanente. Cette période de quatre mois correspond à une absence d'immunité chez le foetus. Lorsque le système immunitaire devient ensuite compétent, celui-ci reconnaît le virus comme sien et il s'établit une situation d'immunotolérance (absence d'anticorps). Ces animaux ne pourront pas survivre à une infection ultérieure par une souche cytopathogène de virus BVD homologue.

Le maintien du virus non cytopathogène au sein de la population bovine est assuré par sa lente dissémination faisant suite à l'infection aiguë d'animaux séronégatifs et, surtout, par son excrétion continuelle par les animaux présentant une virémie persistante (Voir J. Brownlie et al., Ann. Rech. Vét. (1987) 18:157-166).

A. Fenton et al. (Journal of Virological Methods, 27 (1990), 253-260) détectent les antigènes de Pestivirus dans le sang de moutons virémiques infectés de manière persistante par le virus de la maladie de Border, par un ELISA conduit de façon à détecter un antigène spécifique dans les leucocytes de ces animaux. Cette technique nécessite au préalable une purification des leucocytes qui s'avère longue et complexe à réaliser.

Le génome de la souche virale Osloss de biotype cytopathogène a été cloné et entièrement séquencé par Renard et al. (demande de brevet EP-A-0.208.672 du 8 juillet 1985). La demanderesse a trouvé que la phase de lecture ouverte (ORF), de la séquence génomique de BVD Osloss longue de 12408 nucléotides, a une capacité codante de 3951 acides aminés (aas).

Dans un résumé distribué au cours du Symposium sur les infections des ruminants par les Pestivirus qui s'est tenu à Hanovre les 8 et 9 juin 1990, C. Lecomte et al. indiquent l'identification d'un produit de traduction d'ADNc du virus BVD immunoprécipité par des anticorps monoclonaux reconnaissant la protéine non structurale p80 d'un certain nombre de souches de Pestivirus. Le même ADNc est exprimé dans E. coli et l'antigène produit est utilisé en ELISA de compétition pour détecter des anticorps anti-BVD dans le sérum bovin.

La préparation et la caractérisation d'une série d'anticorps monoclonaux ont été décrites par C. Lecomte et al. (Veterinary Microbiology, 23 (1990), 193-201), de même que l'utilisation d'une protéine fusion produite dans E. coli, comme antigène recombinant permettant la détection d'anticorps sériques anti-BVD dans un ELISA de compétition avec les anticorps monoclonaux choisis.

Dans un résumé distribué au VIIIème Congrès international de virologie qui s'est tenu à Berlin du 26 au 31 août 1990, C. Lecomte et al. proposent de recourir à deux tests ELISA pour détecter d'une part les anticorps anti-BVD et d'autre part les antigènes viraux. Les anticorps anti-BVD dans le sérum seraient détectés par un ELISA de compétition mettant en oeuvre un antigène recombinant p80 de BVD Osloss produit dans E. coli et des anticorps monoclonaux dirigés spécifiquement contre la protéine p80 d'un certain nombre de souches de Pestivirus. Le deuxième ELISA serait un ELISA de type "sandwich" utilisant deux anticorps monoclonaux et permettrait la détection d'antigènes chez les animaux virémiques persistants.

La déposante a cependant trouvé que la protéine p80 produite dans E. coli n'est pas reconnue par tous les anticorps monoclonaux anti-p80 et notamment par certains d'entre eux qui présentent une polyspécificité, c'est-à-dire réagissent vis-à-vis de plusieurs ou de toutes les souches de Pestivirus, ce qui compromet les chances de pouvoir détecter en une fois toute infection par une souche quelconque de BVD, d'autant plus que le deuxième ELISA, basé sur l'utilisation de deux anticorps monoclonaux, pourrait aussi ne pas être suffisamment polyspécifique.

Par ailleurs, bien que le génome entier de la souche Osloss ait été sequencé, aucune indication concernant la sequence nucleotidique complète codant pour l'antigène p80 n'etait disponible.

L'invention a pour objectif de fournir un procédé de détection très sensible permettant un contrôle complet et efficace de l'infection éventuelle d' un élevage par un virus BVD quel qu'il soit, et portant à la fois sur la détection des virémies persistantes et des infections aiguës.

L'invention a donc pour objet un procédé de détection de l'infection d'un prélèvement de sang par le virus BVD, comprenant un premier test pour la détection des anticorps anti-BVD et un deuxième test pour la détection des particules virales, caractérisé en ce que l'on détecte les anticorps anti-p80 à l'aide d'un antigène recombinant comprenant la protéine non structurale p80 du virus BVD, produite en hôte eucaryote, et de préférence d'un anticorps monoclonal anti-p80 utilisé comme anticorps compétiteur, et que l'on détecte la présence de particules virales à l'aide d'anticorps, polyclonaux ou monoclonaux, dirigés contre la protéine p80 du virus BVD, et de préférence d'un sérum dirigé contre l'antigène p80 recombinant produit en hôte eucaryote ou procaryote, pour la détection des virémies persistantes et des infections aiguës par n'importe quelle souche de BVD.

L'invention a également pour objet un procédé de détection des anticorps anti-BVD dans un prélèvement de sang, caractérisé en ce que l'on détecte les anticorps anti-p80 à l'aide d'un antigène recombinant comprenant la protéine p80 du virus BVD produite en hôte eucaryote.

La protéine p80 est de préférence issue de BVD Osloss et la séquence nucléotidique codant pour cette protéine à été entièrement séquencée. La séquence est donnée dans la liste de séquences annexée, sous la référence SEQ ID NO:1. La demanderesse a ainsi avantageusement localisé deux sites potentiels de clivage de la p80 (KVR : Lysine - Valine - Arginine) correspondant au début et à la fin de p80.

La protéine p80 est exprimée dans des vecteurs viraux ou eucaryotes et notamment dans le système Baculovirus, lequel est avantageusement le baculovirus AcNPV (Autographa californica nuclear polyhedrosis virus).

La séquence nucléotidique codant pour p80 est introduite dans un vecteur d'expression approprié selon les techniques connues de construction de ces vecteurs, notamment celles décrites dans la demande de brevet EP-A-0.208.672.

Bien entendu, la séquence de nucléotides précitée inclut toutes les séquences équivalentes, c'est-à-dire qui reprennent les propriétés essentielles de la séquence. A titre d'exemple, ce serait le cas d'une séquence qui code pour une séquence identique d'acides aminés, mais qui utiliserait d'autres codons spécifiques par dégénérescence du code. Ce serait également le cas pour une séquence codant pour une séquence d'acides aminés non plus identique mais similaire, compte tenu des ressemblances entre acides aminés.

On entend aussi par séquence équivalente une séquence, codant pour la p80, issue d'une autre souche de BVD et conservant la reconnaissance par les anticorps anti-p80.

L'antigène recombinant lui-même provient de cultures de cellules hôtes eucaryotes qui ont été transfectées par le vecteur exprimant p80, et est constitué de préférence d'extraits de ces cellules. Les hôtes eucaryotes peuvent être des cultures cellulaires animales ou des levures, notamment Saccharomyces cerevisiae. Les vecteurs de transfert pour les levures comportent avantageusement des marqueurs permettant la sélection des recombinants utiles, par exemple par résistance aux antibiotiques ou autres moyens de sélection connus (Broach J. et al., Meth. Enz. (1983) 101 : 307). Pour les promoteurs, voir également Hess et al., J. Adv. Enz. Reg. (1968) 7 / 149, et Holland et al., Biochemistry (1968) 17 : 4900 ou Itzeman et al., J. Biol. Chem. (1980) 255 : 2073.

Les cellules animales sont, de préférence, des lignées cellulaires de mammifères connues telles que HeLa, CHO, ou BHK, des cellules d'insectes, par exemple Spodoptera frugiperda (dépôt ATCC CRL 1711, Sf9) (notamment pour le système Baculovirus) et, d'une façon générale, on préférera des lignées dont l'utilisation pour l'expression de substances à administrer à l'animal a été reconnue par les autorités sanitaires. On utilisera comme promoteur, dans ces constructions cellulaires, des promoteurs viraux tels que ceux du virus SV40 (Fiers et al., Nature, (1978) 273 : 113) et du virus CMV ou cytomegalovirus humain (McGregor et Caskey, Nucleic Acids Res. 17 : 2365, 1989), ou encore celui du gène polyhédrine du Baculovirus AcNPV ou Autographa californica nuclear polyhedrosis virus (Hooft van Iddekinge et al., 1983, virology 131 : 561-565).

L'antigène recombinant est de préférence immobilisé sur un support solide (plaques de microtitrage par exemple), notamment par l'intermédiaire d'un anticorps monoclonal anti-p80 utilisé comme capteur. Des dilutions de sérums bovins sont mises en contact avec l'antigène immobilisé ou non et les anticorps anti-BvD sont soit directement révélés par un anti-sérum anti-IgG bovines couplé par exemple à la peroxydase ou à la biotine (ELISA indirect), soit révélés par un ELISA de compétition avec un deuxième anticorps monoclonal anti-p80 couplé par exemple à la peroxydase ou à la biotine. En fait, cette détection peut être faite sur toute fraction de sang bovin, notamment sérum et plasma.

De préférence, on révèle les particules virales dans le sang complet, brut ou enrichi en globules blancs par une simple centrifugation, notamment pendant 30 minutes à 2500 g.

Pour assurer une détection complète de la présence de particules virales de tous les types de BVD, on utilise de préférence comme capteur un mélange de trois anticorps monoclonaux p80-spécifiques plutôt qu'un seul anticorps monoclonal.

On effectue de préférence un ELISA de type "sandwich" avec, comme capteur, le mélange de trois anticorps monoclonaux spécifiques de la p80 virale et, comme révélateur, le sérum dirigé contre la protéine p80 recombinante. Le sérum provient notamment de l'immunisation d'animaux, notamment de lapins ou de chèvres, par inoculations répétées de p80 recombinante, laquelle peut être produite aussi bien en cellules procaryotes qu'eucaryotes.

On peut ainsi détecter comme positifs des échantillons correspondant à un titre viral inférieur par exemple à 10³ ufp/ml (ufp = unités formatrices de plaques). L'invention permet en fait de détecter comme positifs des échantillons qui, par la méthode usuelle d'immunofluorescence sur cellules infectées, peuvent nécessiter trois passages successifs du virus et le choix des cellules hôtes adéquates, ce gui oblige à propager le virus sur plusieurs types cellulaires.

La protéine p80 ne contenant pas d'épitopes de neutralisation, ce procédé permettra avantageusement de faire la distinction entre les animaux infectés naturellement et les animaux vaccinés par un vaccin recombinant basé sur les protéines structurales du virus.

L'invention a aussi pour objet la séquence de nucléotides référencée SEQ ID NO:1 correspondant à la séquence de BVD Osloss codant pour la protéine non structurale p80, ou une séquence équivalente selon la définition donnée plus haut, ainsi que toute nouvelle séquence nucléotidique qui la contient et qui comporte des moyens permettant son expression ou qui soit associée à de tels moyens.

L'invention a encore pour objet la protéine p80 recombinante correspondant à la traduction de cette séquence, notamment en hôte eucaryote, dans les systèmes d'expression sus-indiqués, et tout antigène recombinant contenant cette séquence, notamment constitué d'extraits de cellules hôtes, en particulier eucaryotes, comme cela a été exposé ci-dessus.

### BREVE DESCRIPTION DU DESSIN.

La figure 1 montre la position des clones d'ADNc couvrant l'ensemble du génome du virus BVD (souche Osloss).
La figure 2 montre l'analyse in vitro de la région du génome de BVD Osloss correspondant au clone d'ADNc 174. Les chiffres y indiquent la taille (paires de bases = pb) des différents fragments obtenus par digestion de l'insert 174 par chacune des enzymes de restriction utilisées.
La figure 3 montre la localisation, sur le génome de BVD Osloss, de la région immunoréactive de 80 acides aminés et des limites de la protéine p80.
La figure 4 montre le criblage d'épitopes dans la région immunoréactive, longue de 80 acides aminés, située au sein de la protéine p80.
La figure 5 montre le schéma du vecteur de transfert pAcYM1 décrit par Matsuura et al. dans J. Gen. Virol. (1987), 68 : 1233 à 1250.

### I - LOCALISATION ET SEQUENCAGE DE LA SEQUENCE CODANT POUR p80.

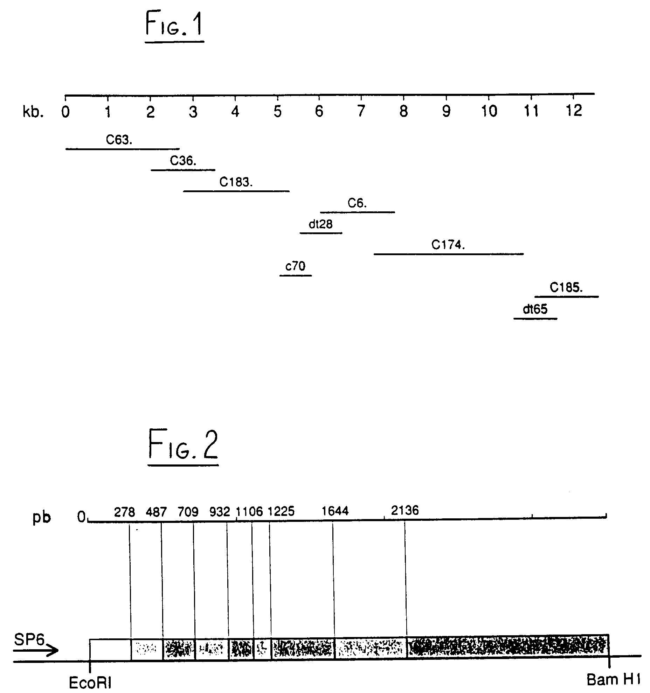

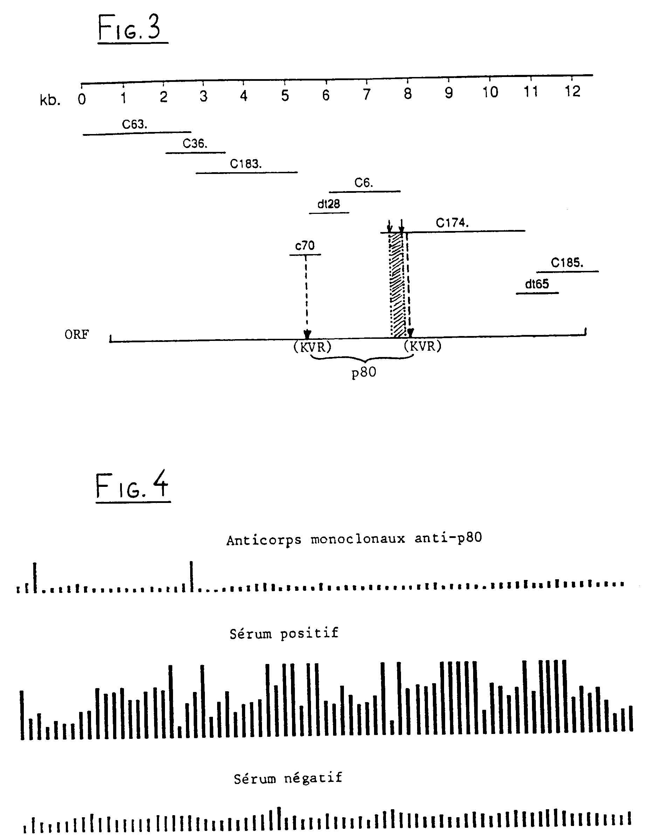

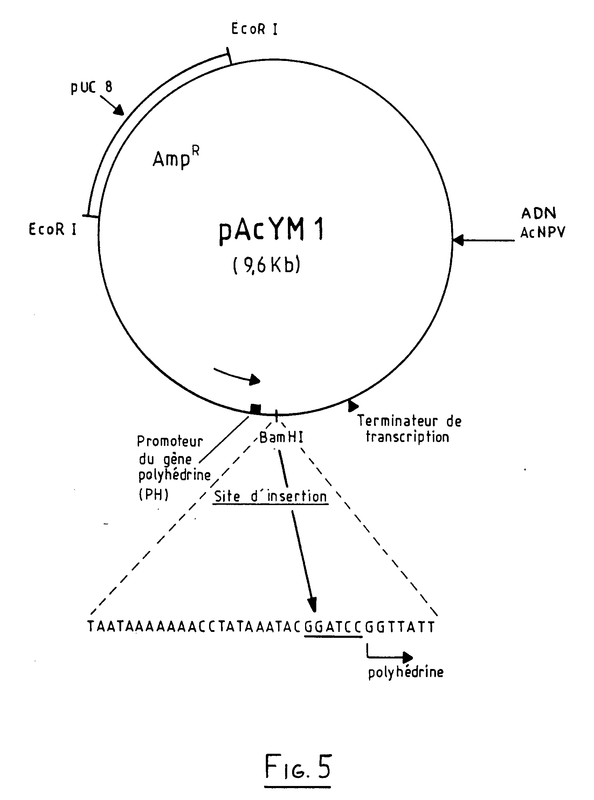

1. On a cloné de l'ADNc C174 (clone pCP174 de la bibliothèque d'ADNc décrite dans la demande de brevet européen EP-A-0.208.672) (figure 1) dans le plasmide pSP65, en aval du promoteur pour l'ARN polymérase du bactériophage SP6, entre les sites EcoRI et BamHI (vecteur décrit par Melton D.A. et al., 1984, Nucleic Acids Res. 12:7035-7056).
2. Après digestion par différentes enzymes de restriction dont la position des sites successifs est indiquée à la figure 2, chaque fragment obtenu a été transcrit in vitro, puis traduit dans un système acellulaire (lysat de réticulocytes de lapin). Les produits de traduction de poids moléculaire croissant ont été immunoprécipités par des anticorps monoclonaux anti-p80 ; cela a permis de localiser une région immunoréactive longue de 80 acides aminés, localisée sur la figure 3 et dont la séquence est donnée dans la liste de séquences sous la référence SEQ ID NO:2.
3. La présence d'épitopes reconnus dans cette région de 80 acides aminés a été confirmée par criblage d'épitopes ("epitope scanning"), c'est-à-dire que 75 hexamères peptidiques recouvrant cette région ont été synthétisés sur support solide et testés en ELISA en présence d'anticorps monoclonaux anti-p80 et de sérums bovins positifs ou négatifs en anticorps. Le résultat est illustré sur la figure 4.
4. Suite à la localisation des épitopes anti-p80, on a reconstitué la séquence codant pour la protéine p80 afin de se rapprocher le plus possible de la protéine naturelle. Par analyse de la séquence génomique du virus BVD/Osloss et comparaison avec celle des Flaviviridae connus, on a déterminé la présence de deux sites potentiels de clivage (KVR : Lysine - Valine - Arginine) correspondant au début et à la fin de la p80 ; le poids moléculaire théorique de la protéine comprise entre ces deux sites est de 80430 daltons. Le premier des deux triplets se retrouve exactement au même endroit dans le génome du virus BVD/NADL, et le génome entier de BVD/Osloss ne contient que 5 triplets KVR, tous situés dans la partie codant pour les protéines non structurales du virus (le génome NADL en contient seulement trois).

Le fragment p80 de 2200 pb (paires de bases) correspondant à la séquence codant pour la p80 a été amplifié par PCR (Polymerase Chain Reaction) et cloné ; sa localisation est illustrée sur la figure 3 ; sa séquence nucléotidigue est référencée SEQ ID NO:1.

### II -EXPRESSION DE L'ANTIGENE RECOMBINANT

L'intégration du fragment p80 dans le génome du Baculovirus Autographa Californica nucléar polyhedrosis virus (AcNPV) a été réalisée par l'intermédiaire du vecteur de transfert pAcYM1 décrit par Matsuura et al. (J. Gen. Virol. 68 : 1233-1250, 1987 ; carte de restriction à la figure 5), en aval du promoteur du gène de la polyhédrine de AcNPV. Ce vecteur contient le site de polyadénylation du gène polyhédrine ainsi que le gène de résistance à l'ampicilline et l'origine de réplication du plasmide pUC8 (J. Messing, 1983, Meth - Enzymol. 101:20).

A cet effet, le fragment amplifié de séquence SEQ ID NO:1 est digéré par les enzymes de restriction NcoI et BamHI, puis traité à l'ADN-polymérase (fragment de Klenow), de manière à rendre franches les extrémités 5′ et 3′ du fragment. On le clone ensuite dans le vecteur pAcYM1 digéré par BamHI et traité à l'ADN-polymérase (fragment de Klenow) pour rendre les extrémités franches. On obtient le plasmide recombinant pAcYM1-p80, la p80 étant exprimée sous la dépendance du promoteur du gène de la polyhédrine.

Le plasmide obtenu est utilisé pour cotransfecter les cellules d'insectes Sf9 en même temps que l'ADN purifié de l'AcNPV type sauvage. Un virus recombinant AcNPV-p80 a été purifié par étalement du surnageant de cotransfection et isolement des plages virales par recouvrement d'agarose.

### III - TEST ELISA DE DETECTION D'ANTICORPS ANTI-p80.

### Principe :

Le test est basé sur la détection des anticorps anti-p80, autrement dit des anticorps dirigés contre une protéine non structurale de 80 000 daltons associée à l'infection par le virus BVD.

Un antigène recombinant comprenant la protéine p80 issue du virus Osloss est fixé sur support solide par l'intermédiaire d'un anticorps monoclonal utilisé comme capteur. L'échantillon de sang ou de sérum bovin est mis en contact avec l'antigène fixé et est ensuite soit directement révélé par un antisérum anti-IgG bovines couplé, soit mis en compétition avec un deuxième anticorps monoclonal anti-p80 couplé.

### Matériel, réactifs et échantillons :

### 1. Matériel et tampons :

- plaques pour ELISA à 96 puits (NUNC - MAXISORP) ;
- tampon carbonate pH 9,6 : Na₂CO₃ 15mM, NaHCO₃ 35mM, NaN₃ 3mM ;
- tampons de lavage : (1) PBS, (2) PBS, Tween® 20 0,1% ;
- tampon de dilution : PBS, Tween® 20 0,1%, sérum de cheval 10% (contrôlé exempt de virus BVD) ;
- tampon de saturation : PBS, sérum de cheval 10% (contrôlé exempt de virus BVD).
- chromogène et tampon adéquat.

### 2. Réactifs :

- 1 anticorps monoclonal anti-p80 utilisé comme capteur de l'antigène p80 (liquide d'ascite dilué) ;
- antigène p80 recombinant ;
- a) ELISA compétition : 1 anticorps monoclonal anti-p80 dilué et couplé à la peroxydase ou à la biotine ;
- b) ELISA indirect : sérum (lapin ou chèvre) anti-IgG bovines couplé à la peroxydase ou à la biotine).

### 3. Préparation des échantillons :

- échantillons sériques : prélèvement du sang en tubes sans anti-coagulant ; coagulation minimum 4 heures à température ambiante ; centrifugation 2500 g, 30 minutes ; prélever le surnageant = sérum.
- le test peut être réalisé sur n'importe quelle fraction sanguine, notamment le plasma ou même le sang complet prélevé en tubes avec anti-coagulant.

### Méthode :

### 1. Fixation du capteur :

L'anticorps monoclonal anti-p80 dilué est réparti à raison de 100 µl par cupule :
   - contact une nuit à 4°C ;
   - 3 lavages avec le tampon (1).

### 2. Saturation (facultative) :

150 µl/cupule de tampon de saturation :
   - 1 heure à 37°C.

### 3. Fixation de l'antigène :

antigène p80 recombinant (100 µl/cupule)
   - contact une nuit à 4°C.

### 4. Contact avec les échantillons :

100 µl/cupule ; sérums dilués 10x :
   - contact 2 heures à 37°C.
   - 3 lavages avec le tampon (1).

### 5. Contact avec l'anticorps révélateur :

a) ELISA compétition : contact avec l'anticorps monoclonal dilué et couplé :
   - 1h30 à 37°C ;
   - 3 lavages avec le tampon (2) ;
   - 3 lavages à l'eau.
b) ELISA indirect : contact avec le sérum anti-IgG bovines dilué et couplé :
   - 1h30 à 37°C ;
   - 3 lavages avec le tampon (2) :
   - 3 lavages à l'eau.

### 6. Révélation par le chromogène :

La procédure varie suivant le chromogène et est détaillée par le fournisseur de celui-ci.

### IV - TEST ELISA DE DETECTION DE PARTICULES VIRALES.

### Principe :

Le test est basé sur la détection de la protéine p80, protéine non structurale du virus BVD présente en grande quantité dans le sang des animaux infectés. Un mélange de trois anticorps monoclonaux spécifiques de cette protéine p80 est utilisé comme capteur et fixé sur support solide (plaques de microtitrage). Le sans complet ou la fraction de sang enrichie en globules blancs ou "buffy coat" est mise en contact avec le mélange capteur fixé. Un sérum (lapin ou chèvre) dirigé contre la protéine p80 recombinante est utilisé comme anticorps révélateur, soit directement couplé (à la peroxydase ou à la biotine), soit révélé lui-même par un deuxième anticorps (anti-IgG de lapin ou de chèvre) couplé à la peroxydase ou à la biotine.

La présence d'antigène viral dans les échantillons testés se traduit par l'élévation de la densité optique de base en présence de chromogène.

### Matériel, réactifs et échantillons :

### 1. Matériel et tampons :

- plaques pour ELISA à 96 puits : NUNC - MAXISORP.
- tampon carbonate pH 9,6 : Na₂CO₃ 15 mM, NaHCO₃ 35 mM, NaN₃ 3 mM.
- tampon de lavage : PBS, Tween® 20 0,1 %.
- tampon de saturation/dilution : PBS, Tween® 20 0,1 %, sérum de cheval 10 % (contrôlé exempt de virus BVD).
- chromogène et tampon adéquat.

### 2. Réactifs :

- mélange de 3 anticorps monoclonaux anti-p80, liquides d'ascite dilués.
- sérum (lapin ou chèvre) dirigé contre la protéine p80 recombinante, soit directement couplé (à la peroxydase ou à la biotine), soit révélé lui-même par un deuxième anticorps (anti-IgG de lapin ou de chèvre) couplé à la peroxydase ou à la biotine.

### 3. Préparation des échantillons :

Prélèvement du sans en tubes héparinés ; les échantillons sont testés soit tels quels, soit après enrichissement en globules blancs suivant la procédure suivante : centrifugation à 2500 g ; 30 minutes ; après centrifugation, éliminer par pipettage la couche supérieure constituée par le plasma et prélever le "buffy coat", zone blanchâtre située entre le plasma et les globules rouges.

### Méthode :

### 1. Fixation du capteur :

Mélange d'anticorps monoclonaux dilués et répartis à raison de 100 µl par cupule.
   - Contact une nuit à 4°C.
   - 3 lavages en PBS/Tween®.

### 2. Saturation :

150 µl/cupule de tampon de saturation contenant 10 % de sérum de cheval.
   - 1 heure à 37°C.

### 3. Contact avec les échantillons :

100 µl de sang complet ou de "buffy coat" par cupule.
   - Contact 2 heures à 37°C.
   - 3 lavages en PBS/Tween.

### 4. Contact avec l'anticorps révélateur :

Sérum anti-p80 dilué, couplé ou non (peroxydase ou biotine) :
   - contact 1 h à 37°C (100 µl/cupule) ;
   - 3 lavages en PBS/Tween® ;
   - 3 lavages à l'eau ;
En cas d'utilisation de sérum non couplé, à nouveau contact avec antisérum couplé (1 heure à 37°C).

### 5. Révélation par le chromogène :

La procédure varie suivant le chromogène choisi et est détaillée par le fournisseur de celui-ci.

LISTE DES SEQUENCES.

SEQ ID NO : 1

TYPE DE SEQUENCE : séquence nucléotidique

LONGUEUR DE LA SEQUENCE : 2236 paires de bases

NOMBRE DE BRINS : simple

CONFIGURATION : linéaire

TYPE DE MOLECULE : ADNc pour ARN génomique

ORIGINE : BVD Osloss

REGION TRADUITE : 40 - 2229

PROPRIETES : code pour la protéine p80

Met Gly Leu 3
TCT AGA AAT TTT GTT TAA CTT TAA GAA GGA GAT ATA CCC ATG GGA CTG 48
XbaI NcoI

Glu Thr Gly Trp Ala Tyr Thr His Gln Gly Gly Ile Ser Ser Val Asp 19
GAA ACC GGA TGG GCC TAC ACA CAC CAA GGC GGC ATA AGC TCA GTA GAC 96

His Val Thr Ala Gly Lys Asp Leu Leu Val Cys Asp Ser Met Gly Arg 35
CAT GTG ACC GCA GGC AAA GAC CTA CTG GTT TGT GAT AGT ATG GGT AGG 144

Thr Arg Val Val Cys Gln Ser Asn Asn Lys Leu Thr Asp Glu Thr Glu 51
ACA AGA GTG GTT TGC CAA AGT AAC AAC AAG TTA ACT GAT GAG ACA GAA 192

Tyr Gly Val Lys Thr Asp Ser Gly Cys Pro Asp Gly Ala Arg Cys Tyr 67
TAT GGT GTC AAG ACG GAC TCC GGA TGT CCA GAT GGT GCC AGG TGC TAC 240

Val Leu Asn Pro Glu Ala Val Asn Ile Ser Gly Ser Lys Gly Ala Ala 83
GTA TTA AAT CCA GAG GCA GTA AAT ATA TCA GGG TCC AAG GGA GCT GCT 288

Val His Leu Gln Lys Thr Gly Gly Glu Phe Thr Cys Val Thr Ala Ser 99
GTA CAC CTC CAA AAA ACA GGT GGG GAA TTC ACA TGT GTT ACT GCA TCG 336

Gly Thr Pro Ala Phe Phe Asp Leu Lys Asn Leu Lys Gly Trp Ser Gly 115
GGA ACT CCA GCC TTC TTT GAC CTG AAA AAT TTG AAG GGA TGG TCA GGT 384

Leu Pro Ile Phe Glu Ala Ser Ser Gly Arg Val Val Gly Arg Val Lys 131
CTA CCC ATA TTT GAG GCT TCT AGT GGC AGG GTG GTC GGC AGA GTT AAA 432

Val Gly Lys Asn Glu Glu Ser Lys Pro Thr Lys Leu Met Ser Gly Ile 147
GTA GGA AAG AAT GAG GAA TCC AAG CCC ACA AAA TTA ATG AGT GGT ATC 480

Gln Thr Val Ser Lys Ser Thr Ala Asp Leu Thr Glu Met Val Lys Lys 163
CAA ACC GTC TCA AAA AGC ACA GCC GAT TTA ACA GAG ATG GTC AAG AAG 528

Ile Thr Ser Met Asn Arg Gly Asp Phe Lys Gln Ile Thr Leu Ala Thr 179
ATA ACC AGC ATG AAC AGG GGA GAC TTT AAG CAG ATA ACC CTT GCA ACA 576

Gly Ala Gly Lys Thr Thr Glu Leu Pro Lys Ala Val Ile Glu Glu Ile 195
GGG GCA GGA AAA ACT ACA GAA CTC CCA AAG GCA GTG ATA GAG GAG ATA 624

Gly Arg His Lys Arg Val Leu Val Leu Ile Pro Leu Arg Ala Ala Ala 211
GGA AGA CAC AAG CGG GTG CTA GTG CTT ATA CCA TTG AGA GCA GCA GCT 672

Glu Ser Val Tyr Gln Tyr Met Arg Leu Lys His Pro Ser Ile Ser Phe 227
GAG TCA GTC TAT CAA TAC ATG AGA TTG AAA CAT CCC AGT ATC TCC TTC 720

Asn Leu Arg Ile Gly Asp Met Lys Glu Gly Asp Met Ala Thr Gly Ile 243
AAC TTA AGA ATA GGG GAC ATG AAA GAA GGG GAC ATG GCA ACT GGG ATC 768

Thr Tyr Ala Ser Tyr Gly Tyr Phe Cys Gln Met Pro Gln Pro Lys Leu 259
ACC TAC GCC TCA TAT GGA TAT TTT TGC CAA ATG CCG CAG CCG AAG CTC 816

Arg Ala Ala Met Val Glu Tyr Ser Tyr Ile Phe Leu Asp Glu Tyr His 275
AGG GCC GCA ATG GTA GAG TAT TCA TAC ATA TTT CTG GAT GAG TAT CAC 864

Cys Ala Thr Pro Glu Gln Leu Ala Val Ile Gly Lys Ile His Arg Phe 291
TGT GCT ACT CCT GAG CAG TTG GCT GTC ATA GGA AAA ATT CAC AGA TTT 912

Ser Glu Ser Ile Arg Val Val Ala Met Thr Ala Thr Pro Ala Gly Ser 307
TCT GAA AGC ATA AGG GTG GTT GCT ATG ACC GCC ACC CCA GCA GGG TCA 960

Val Thr Thr Thr Gly Gln Lys His Pro Ile Glu Glu Phe Ile Ala Pro 323
GTA ACT ACA ACA GGG CAA AAA CAC CCA ATA GAA GAA TTC ATA GCT CCT 1008

Glu Val Met Lys Gly Glu Asp Leu Gly Ser Gln Phe Leu Asp Ile Ala 339
GAG GTG ATG AAA GGG GAA GAC CTT GGA AGC CAG TTC CTT GAC ATA GCG 1056

Gly Leu Lys Ile Pro Val Glu Glu Met Lys Gly Asn Met Leu Val Phe 355
GGG CTA AAA ATC CCG GTT GAG GAG ATG AAG GGT AAC ATG CTG GTC TTC 1104

Val Pro Thr Arg Asn Met Ala Val Asp Val Ala Lys Lys Leu Lys Ala 371
GTA CCC ACA AGA AAC ATG GCA GTT GAT GTA GCC AAG AAA CTA AAA GCC 1152

Lys Gly Tyr Asn Ser Gly Tyr Tyr Tyr Ser Gly Glu Asp Pro Ala Asn 387
AAG GGC TAC AAC TCA GGG TAT TAC TAC AGT GGG GAA GAC CCG GCT AAC 1200

Leu Arg Val Val Thr Ser Gln Ser Pro Tyr Val Val Val Ala Thr Asn 403
TTG AGG GTG GTA ACA TCA CAG TCC CCA TAC GTC GTA GTA GCC ACC AAT 1248

Ala Ile Glu Ser Gly Val Thr Leu Pro Asp Leu Asp Thr Val Val Asp 419
GCC ATT GAG TCA GGG GTA ACG CTG CCA GAT TTA GAT ACA GTT GTT GAC 1296

Thr Gly Leu Lys Cys Glu Lys Arg Val Arg Val Ser Ser Lys Ile Pro 435
ACA GGT CTG AAG TGT GAA AAG AGG GTG AGG GTG TCA TCA AAA ATA CCT 1344

Phe Ile Val Thr Gly Leu Lys Arg Met Ala Val Thr Val Gly Glu Gln 451
TTC ATA GTA ACA GGC CTT AAA AGA ATG GCT GTC ACT GTG GGC GAA CAG 1392

Ala Gln Arg Arg Gly Arg Val Gly Arg Val Lys Pro Gly Arg Tyr Tyr 467
GCT CAG CGA AGA GGC AGG GTA GGT AGA GTG AAG CCC GGT AGG TAC TAT 1440

Arg Ser Gln Glu Thr Ala Thr Gly Ser Lys Asp Tyr His Tyr Asp Leu 483
AGA AGC CAG GAA ACA GCG ACC GGG TCA AAG GAC TAC CAC TAT GAC CTG 1488

Leu Gln Ala His Arg Tyr Gly Ile Glu Asp Gly Ile Asn Val Thr Lys 499
TTA CAG GCA CAC AGG TAT GGG ATA GAA GAT GGA ATC AAC GTG ACA AAG 1536

Ser Phe Arg Glu Met Asn Tyr Asp Trp Ser Leu Tyr Glu Glu Asp Ser 515
TCC TTT AGG GAA ATG AAT TAC GAT TGG AGC CTG TAC GAG GAG GAC AGC 1584

Leu Leu Ile Thr Gln Leu Glu Ile Leu Asn Asn Leu Leu Ile Ser Glu 531
TTG CTG ATA ACG CAG CTG GAG ATA CTG AAC AAT CTA CTC ATC TCT GAA 1632

Asp Leu Pro Ala Ala Val Lys Asn Ile Met Ala Arg Thr Asp His Pro 547
GAC CTA CCA GCA GCA GTA AAA AAC ATC ATG GCA AGG ACT GAT CAC CCA 1680

Glu Pro Ile Gln Leu Ala Tyr Asn Ser Tyr Glu Val Gln Val Pro Val 563
GAA CCA ATC CAG CTT GCA TAC AAC AGT TAT GAG GTC CAG GTC CCT GTA 1728

Leu Phe Pro Lys Ile Arg Asn Gly Glu Val Thr Asp Thr Tyr Glu Asn 579
CTG TTT CCA AAA ATA AGG AAT GGG GAG GTT ACA GAT ACT TAC GAG AAC 1776

Tyr Ser Phe Leu Asn Ala Arg Lys Leu Gly Glu Asp Val Pro Val Tyr 595
TAC TCA TTC CTA AAT GCA AGA AAA CTA GGG GAA GAT GTA CCT GTG TAC 1824

Ile Tyr Ala Thr Glu Asp Glu Asp Leu Ala Val Asp Leu Leu Gly Leu 611
ATT TAT GCC ACC GAA GAT GAA GAC CTG GCA GTA GAC CTT CTA GGC TTG 1872

Asp Trp Pro Asp Pro Gly Asn Gln Gln Val Val Glu Thr Gly Lys Ala 627
GAC TGG CCC GAC CCA GGG AAC CAG CAA GTA GTG GAG ACT GGG AAA GCA 1920

Leu Lys Gln Val Val Gly Leu Ser Ser Ala Glu Asn Ala Leu Leu Ile 643
CTG AAG CAA GTG GTA GGA CTG TCC TCT GCT GAG AAT GCC CTG CTC ATA 1968

Ala Leu Phe Gly Tyr Val Gly Tyr Gln Ala Leu Ser Lys Arg His Val 659
GCC CTG TTT GGG TAT GTA GGA TAT CAA GCT TTG TCA AAA AGA CAC GTC 2016

Pro Met Ile Thr Asp Ile Tyr Thr Ile Glu Asp Gln Arg Leu Glu Asp 675
CCA ATG ATC ACA GAC ATA TAC ACC ATA GAA GAT CAA AGA CTA GAG GAC 2064

Thr Thr His Leu Gln Tyr Ala Pro Asn Ala Ile Arg Thr Glu Gly Lys 691
ACA ACC CAC CTC CAA TAT GCA CCT AAT GCT ATA AGA ACT GAG GGG AAG 2112

Glu Thr Glu Leu Lys Glu Leu Ala Val Gly Asp Met Asp Arg Ile Met 707
GAG ACT GAA CTA AAG GAA TTA GCA GTG GGT GAC ATG GAC AGA ATC ATG 2160

Glu Ser Ile Ser Asp Tyr Ala Ser Gly Gly Leu Thr Phe Ile Arg Ser 723
GAA TCC ATC TCA GAT TAT GCA TCA GGA GGG TTG ACA TTC ATA AGA TCT 2208

Gln Ala Glu Lys Val Arg STOP *** 729
CAG GCA GAG AAA GTA CGC TAG CGG ATC C 2236

BamHI

SEQ ID NO : 2
TYPE DE SEQUENCE : séquence nucléotidique
LONGUEUR DE LA SEQUENCE : 240 paires de bases
NOMBRE DE BRINS : simple
CONFIGURATION : linéaire
TYPE DE MOLECULE : ADNc pour ARN génomique
ORIGINE : BVD Osloss
REGION TRADUITE : 1 - 240
PROPRIETES : code pour une région de 80 acides aminés
de la p80, comprenant des épitopes recon-
nus par des anticorps monoclonaux anti-p80.

Gly Tyr Gln Ala Leu Ser Lys Arg His Val Pro Met Ile Thr Asp Ile 16
GGA TAT CAA GCT TTG TCA AAA AGA CAC GTC CCA ATG ATC ACA GAC ATA 48

Tyr Thr Ile Glu Asp Gln Arg Leu Glu Asp Thr Thr His Leu Gln Tyr 32
TAC ACC ATA GAA GAT CAA AGA CTA GAG GAC ACA ACC CAC CTC CAA TAT 96

Ala Pro Asn Ala Ile Arg Thr Glu Gly Lys Glu Thr Glu Leu Lys Glu 48
GCA CCT AAT GCT ATA AGA ACT GAG GGG AAG GAG ACT GAA CTA AAG GAA 144

Leu Ala Val Gly Asp Met Asp Arg Ile Met Glu Ser Ile Ser Asp Tyr 64
TTA GCA GTG GGT GAC ATG GAC AGA ATC ATG GAA TCC ATC TCA GAT TAT 192

Ala Ser Gly Gly Leu Thr Phe Ile Arg Ser Gln Ala Glu Lys Val Arg 80
GCA TCA GGA GGG TTG ACA TTC ATA AGA TCT CAG GCA GAG AAA GTA AGA 240

## Revendications

1. Procédé de détection de l'infection d'un prélèvement de sang par un virus de la diarrhée bovine (BVD), comprenant un premier test pour la détection des anticorps anti-BVD et un deuxième test pour la détection des particules virales, caractérisé en ce que l'on détecte les anticorps anti-p80 à l'aide d'un antigène recombinant comprenant la protéine p80 du virus BVD produite en hôte eucaryote et que l'on détecte la présence de particules virales à l'aide d'anticorps dirigés contre la protéine p80 du virus BVD, pour la détection des virémies persistantes et des infections aiguës par n'importe quelle souche de BVD.

2. Procédé de détection des anticorps anti-BVD dans un prélèvement de sang, caractérisé en ce que l'on détecte les anticorps anti-p80 à l'aide d'un antigène recombinant comprenant la protéine p80 du virus BVD produite en hôte eucaryote.

3. Procédé selon l'une quelconque des revendications 1 ou 2, caractérisé en ce que la protéine p80 recombinante est codée par la séquence nucléotidique SEQ ID N°:1 ou une séquence équivalente.

4. Procédé selon l'une quelconque des revendications 1 à 3, caractérisé en ce que l'antigène p80 recombinant provient de cultures cellulaires animales transfectées par un vecteur convenable exprimant la protéine p80.

5. Procédé selon la revendication 4, caractérisé en ce que l'antigène p80 recombinant provient de cultures de cellules d'insectes, telles que Spodoptera frugiperda.

6. Procédé selon la revendication 4, caractérisé en ce que l'antigène p80 recombinant provient de cultures de cellules de mammifères, telles que Hela, CHO ou BHK.

7. Procédé selon l'une quelconque des revendications 1 à 3, caractérisé en ce que l'antigène p80 recombinant provient de cultures de cellules de levure, telles que Saccharomyces cerevisiae, transfectées par un vecteur convenable exprimant la protéine p80.

8. Procédé selon l'une quelconque des revendications 1 à 7, caractérisé en ce que l'antigène p80 recombinant comprend des extraits de cellules hôtes eucaryotes transfectées par un vecteur convenable exprimant la protéine p80.

9. Procédé selon l'une quelconque des revendications 1 à 8, caractérisé en ce que la protéine p80 est obtenue par expression dans un vecteur viral ou eucaryote.

10. Procédé selon la revendication 9, caractérisé en ce que la protéine p80 est exprimée dans le système Baculovirus, tel que le baculovirus AcPNV.

11. Procédé selon l'une quelconque des revendications 1 à 10, caractérisé en ce que l'on détecte les anticorps anti-p80 dans toute fraction de sang, sérum ou plasma.

12. Procédé selon l'une quelconque des revendications 1 à 11, caractérisé en ce que l'antigène p80 recombinant permettant la détection d'anticorps est immobilisé sur un support solide.

13. Procédé selon l'une quelconque des revendications 1 à 12, caractérisé en ce que l'on révèle la présence d'anticorps anti-p80 par un anti-sérum anti-IgG bovines couplé à un révélateur.

14. Procédé selon l'une quelconque des revendications 1 à 12, caractérisé en ce que l'on révèle la présence d'anticorps par un test de compétition, en présence d'un anticorps monoclonal anti-p80 couplé à un révélateur.

15. Procédé selon l'une quelconque des revendications 1 et 3 à 14, caractérisé en ce que l'on détecte la présence de particules virales dans le sang complet, brut ou enrichi en globules blancs.

16. Procédé selon l'une quelconque des revendications 1 et 3 à 15, caractérisé en ce que l'on détecte la présence de particules virales à l'aide d'un sérum dirigé contre l'antigène p80 recombinant produit en hôte eucaryote ou procaryote.

17. Procédé selon l'une quelconque des revendications 1 et 3 à 16, caractérisé en ce que, pour la détection des particules virales, on utilise un mélange de trois anticorps monoclonaux spécifiques de la p80 virale.

18. Procédé selon l'ensemble des revendications 16 et 17, caractérisé en ce qu'on effectue un test de type "sandwich" avec le mélange d'anticorps monoclonaux comme capteur et le sérum comme révélateur.

19. Polynucléotide de séquence référencée SEQ ID N°:1 et correspondant à la séquence de BVD Osloss codant pour la protéine non structurale p80.

20. Protéine recombinante correspondant à la traduction de la séquence nucléotidique selon la revendication 19.

21. Protéine recombinante selon la revendication 20, caractérisée en ce qu'elle est produite en hôte eucaryote transfecté par un vecteur convenable exprimant ladite séquence nucléotidique.

22. Protéine recombinante selon la revendication 21, caractérisée en ce qu'elle est produite dans des cellules d'insectes Spodoptera frugiperda transfectées par un vecteur Baculovirus, tel que le baculovirus AcNPV.

23. Antigène recombinant contenant la protéine recombinante selon l'une des revendications 20 à 22.

24. Antigène recombinant selon la revendication 23, caractérisé en ce qu'il comprend des extraits de cellules hôtes transfectées par un vecteur exprimant ladite protéine recombinante.

## Claims

1. Process for detecting infection in a blood sample by a bovine diarrhoea virus (BDV), comprising a first test for detecting anti-BDV antibodies and a second test for detecting viral particles, characterised in that the anti-p80 antibodies are detected using a recombinant antigen comprising the protein p80 of the BDV virus produced in a eukaryotic host and the presence of viral particles is detected by means of antibodies directed against the protein p80 of the virus BDV, for detecting persistent viraemias and acute infections by any strain of BDV.

2. Process for detecting anti-BDV antibodies in a blood sample, characterised in that the anti-p80 antibodies are detected by means of a recombinant antigen comprising the protein p80 of the BDV virus produced in a eukaryotic host.

3. Process according to either of claims 1 and 2, characterised in that the recombinant p80 protein is coded by the nucleotide sequence SEQ ID No. 1 or an equivalent sequence.

4. Process according to any one of claims 1 to 3, characterised in that the recombinant p80 antigen originates from animal cell cultures transfected by a suitable vector which expresses the protein p80.

5. Process according to claim 4, characterised in that the recombinant p80 antigen originates from insect cell cultures such as *Spodoptera frugiperda*.

6. Process according to claim 4, characterised in that the recombinant p80 antigen originates from mammalian cell cultures such as Hela, CHO or BHK.

7. Process according to any one of claims 1 to 3, characterised in that the recombinant p80 antigen originates from yeast cell cultures such as *Saccharomyces cerevisiae*, transfected with a suitable vector expressing the protein p80.

8. Process according to any one of claims 1 to 7, characterised in that the recombinant p80 antigen comprises eukaryotic host cell extracts transfected with a suitable vector expressing the protein p80.

9. Process according to any one of claims 1 to 8, characterised in that the protein p80 is obtained by expression in a viral or eukaryotic vector.

10. Process according to claim 9, characterised in that the protein p80 is expressed in the baculovirus system such as the baculovirus AcPNV.

11. Process according to any one of claims 1 to 10, characterised in that the anti-p80 antibodies are detected in any blood, serum or plasma fraction.

12. Process according to any one of claims 1 to 11, characterised in that the recombinant antigen p80 which makes it possible to detect antibodies is immobilised on a solid substrate.

13. Process according to any one of claims 1 to 12, characterised in that the presence of anti-p80 antibodies is revealed by an anti-IgG bovine antiserum coupled with a developer.

14. Process according to any one of claims 1 to 12, characterised in that the presence of antibodies is revealed by a competitive test in the presence of a monoclonal anti-p80 antibody coupled with a developer.

15. Process according to any one of claims 1 and 3 to 14, characterised in that the presence of viral particles is detected in whole blood, untreated blood or blood enriched with white corpuscles.

16. Process according to any one of claims 1 and 3 to 15, characterised in that the presence of viral particles is detected by means of a serum directed against the recombinant p80 antigen produced in a eukaryotic or prokaryotic host.

17. Process according to any one of claims 1 and 3 to 16, characterised in that, for detecting viral particles, a mixture of three monoclonal antibodies specific to the viral p80 is used.

18. Process according to claims 16 and 17 together, characterised in that a "sandwich" type test is carried out with the mixture of monoclonal antibodies as absorber and the serum as the developer.

19. Polynucleotide having the sequence designated SEQ ID No. 1 and corresponding to the Osloss BDV sequence coded for the non-structural protein p80.

20. Recombinant protein corresponding to the translation of the nucleotide sequence according to claim 19.

21. Recombinant protein according to claim 20, characterised in that it is produced in a eukaryotic host transfected with a suitable vector expressing the nucleotide sequence.

22. Recombinant protein according to claim 21, characterised in that it is produced in cells of the insects *Spodoptera frugiperda* transfected by a baculovirus vector such as baculovirus AcNPV.

23. Recombinant antigen containing the recombinant protein according to one of claims 20 to 22.

24. Recombinant antigen according to claim 23, characterized in that it comprises extracts from host cells transfected by a vector expressing said recombinant protein.

## Patentansprüche

1. Verfahren zum Nachweis der Infektion einer Blutprobe mit einem Rinderdiarrhoevirus (BVD), umfassend einen ersten Test zum Nachweis von anti-BVD-Antikörpern und einen zweiten Test zum Nachweis von Viruspartikeln, dadurch gekennzeichnet, dass man anti-p80-Antikörper mit Hilfe eines rekombinanten Antigens, umfassend das im eukaryotischen Wirt erzeugte Protein p80 des BVD-Virus, nachweist und das Vorliegen von Viruspartikeln mit Hilfe von Antikörpern, die gegen das p80-Protein des BVD-Virus gerichtet sind, nachweist, so dass dauerhafte Virämien und akute Infektionen mit irgendeinen BVD-Stamm nachgewiesen werden.

2. Verfahren zum Nachweis von anti-BVD-Antikörpern in einer Blutprobe, dadurch gekennzeichnet, dass man anti-p80-Antikörper mit Hilfe eines rekombinanten Antigens, umfassend das im eukaryotischen Wirt erzeugte Protein p80 des BVD-Virus, nachweist.

3. Verfahren nach einem der Ansprüche 1 oder 2, dadurch gekennzeichnet, dass das rekombinante p80-Protein von der Nukleotidsequenz SEQ ID NR:1 oder einer äquivalenten Sequenz codiert wird.

4. Verfahren nach einem der Ansprüche 1 bis 3, dadurch gekennzeichnet, dass das rekombinante p80-Antigen aus tierischen Zellkulturen herrührt, die mit einem geeigneten, das p80-Protein exprimierenden Vektor transfiziert sind.

5. Verfahren nach Anspruch 4, dadurch gekennzeichnet, dass das rekombinante p80-Antigen von Insektenzellkulturen, wie Spodoptera frugiperda, herrührt

6. Verfahren nach Anspruch 4, dadurch gekennzeichnet, dass das rekombinante p80-Antigen von Säugerzellkulturen, wie Hela, CHO oder BHK, herrührt.

7. Verfahren nach einem der Ansprüche 1 bis 3, dadurch gekennzeichnet, dass das rekombinante p80-Antigen von Hefezellkulturen, wie Saccharomyces cerevisiae, die mit einem geeigneten, das p80-Protein exprimierenden Vektor transfiziert sind, herrührt.

8. Verfahren nach einem der Ansprüche 1 bis 7, dadurch gekennzeichnet, dass das rekombinante p80-Antigen Extrakte eukaryotischer Wirtszellen, die mit einem geeigneten, das p80-Protein exprimierenden Vektor transfiziert sind, umfasst.

9. Verfahren nach einem der Ansprüche 1 bis 8, dadurch gekennzeichnet, dass das p80-Protein durch Expression in einem viralen oder eukaryotischen Vektor erhalten wird.

10. Verfahren nach Anspruch 9, dadurch gekennzeichnet, dass das p80-Protein im Baculovirussystem, wie Baculovirus AcPNV, exprimiert wird.

11. Verfahren nach einem der Ansprüche 1 bis 10, dadurch gekennzeichnet, dass man anti-p80-Antikörper in jeder Fraktion von Blut, Serum oder Plasma nachweist.

12. Verfahren nach einem der Ansprüche 1 bis 11, dadurch gekennzeichnet, dass das rekombinante p80-Antigen, das den Nachweis von Antikörpern ermöglicht, an einem festen Träger immobilisiert ist.

13. Verfahren nach einem der Ansprüche 1 bis 12, dadurch gekennzeichnet, dass man das Vorliegen von anti-p80-Antikörpern durch ein anti-Rinder-IgG-Antiserum, das mit einer Markierung gekoppelt ist, sichtbar macht.

14. Verfahren nach einem der Ansprüche 1 bis 12, dadurch gekennzeichnet, dass man das Vorliegen von Antikörpern durch einen Kompetitionstest in Anwesenheit monoklonaler anti-p80-Antikörper, gekoppelt an eine Markierung, sichtbar macht.

15. Verfahren nach einem der Ansprüche 1 und 3 bis 14, dadurch gekennzeichnet, dass man das Vorliegen von Viruspartikeln in Vollblut, in unbehandeltem oder in mit weißen Blutzellen angereichertem Blut nachweist.

16. Verfahren nach einem der Ansprüche 1 und 3 bis 15, dadurch gekennzeichnet, dass man das Vorliegen von Viruspartikeln mit einem Serum, das gegen das im eukaryotischen oder prokaryotischen Wirt hergestellte rekombinante p80-Antigen gerichtet ist, nachweist.

17. Verfahren nach einem der Ansprüche 1 und 3 bis 16, dadurch gekennzeichnet, dass man zum Nachweis von Viruspartikeln ein Gemisch von drei für virales p80 spezifischen monoklonalen Antikörpern verwendet.

18. Verfahren nach den Ansprüchen 16 und 17, dadurch gekennzeichnet, dass man einen Test vom "Sandwich"-Typ mit dem Gemisch monoklonaler Antikörper als Fänger und dem Serum als Markierung durchführt.

19. Polynukleotid mit der als SEQ ID NR:1 bezeichneten Sequenz, die der Sequenz von BVD-Osloss entspricht, die das nicht strukturelle Protein p80 codiert.

20. Rekombinantes Protein, das der Translation der Nukleotidsequenz nach Anspruch 19 entspricht.

21. Rekombinantes Protein nach Anspruch 20, dadurch gekennzeichnet, dass es in einem eukaryotischen Wirt, der mit einen geeigneten, diese Nukleotidsequenz exprimierenden Vektor transfiziert ist, hergestellt wird.

22. Rekombinantes Protein nach Anspruch 21, dadurch gekennzeichnet, dass es in Spodoptera frugiperda-Insektenzellen, die mit einem Baculovirusvektor, wie Baculovirus-AcNPV, transfiziert sind, hergestellt wird.

23. Rekombinantes Antigen, das das rekonbinante Protein nach einem der Ansprüche 20 bis 22 umfasst.

24. Rekombinantes Antigen nach Anspruch 23, dadurch gekennzeichnet, das es Extrakte von Wirtszellen, die mit einem das rekombinante Protein exprimierenden Vektor transfiziert sind, umfasst.
